# EUROPEAN PATENT APPLICATION

(11) **EP 4 470 455 A1**
(43) Date of publication of application: **04.12.2024**
(21) Application number: 23176737.7
(22) Date of filing: 01.06.2023
(51) Int. Cl.: A61B 5/024, A61B 5/1455, A61B 5/00

(54) **PHOTOPLETHYSMOGRAPHY SENSOR**

(71) Applicant: Koninklijke Philips N.V., 5656 AG Eindhoven (NL)
(72) Inventor: JOOSTEN, Franciscus Ivo, Eindhoven (NL)
(74) Representative: Philips Intellectual Property & Standards

(57) **Abstract**

The invention provides a PPG sensor system which comprises an optical waveguide, facilitating the transmission of light from a light source to a distal end of the waveguide. In this way, light from the light source can be emitted at a location separate to the location of the said light source. By employing an optical waveguide, light can be emitted in a PPG sensor system at a point non-proximate to the light source. For instance, light can be emitted from the waveguide at a point opposite the light detector and pointed towards it, such that transmissive PPG may be enabled. By using an optical waveguide, the light source and the light detector can be integrated in the same circuit board and housing, thus increasing the efficiency of the PPG sensor system.

## Description

### FIELD OF THE INVENTION

This invention relates to a Photoplethysmography (PPG) sensor system.

### BACKGROUND OF THE INVENTION

PPG measurements have been used for a long time to easily obtain vital signs. First, this was restricted to only obtaining heart rate, but over the years, more vital signs have been able to be extracted from the acquired data. The principle is that a light, typically an LED, emits through a subject's tissue and therefore through blood veins, and a photo diode or light detector placed on the opposite side can capture the transmitted light. This is referred to as transmissive PPG. Based on variations in the amount of light arriving at the photo diode, it can be determined what the subject's heart rate is. Nowadays, with the use of different colored LEDs and advanced algorithms, other vital signs can be acquired such as respiration rate and SpO2. In other words, in a transmissive PPG sensor, the LED and photodiode are positioned on opposite sides of the subject's tissue. Thus, to measure the transmissive PPG signal, the LED light needs to penetrate the human tissue to reach the photodiode on the other side of the tissue.

Integrating LEDs opposite the photodiode is difficult for certain applications, however, such as smart watches where watertightness is important. Therefore, for cost effectiveness and ease of manufacturing, it is desirable that the LED(s) is not opposite the photodiode so that they can be integrated into the same watertight circuit board and housing. To this end, the reflective PPG measurement method has been developed, where variations in the reflections of light bouncing off the subject's veins are acquired and processed with an algorithm. In other words, in a reflective PPG sensor, the LED and the photodiode are positioned on the same side of the subject's tissue and thus the LED light is reflected by the subject's tissue and reaches the photodiode on the same side. This works reasonably for acquiring heart rate and some other vital signs, but it is less accurate and has difficulties, especially, for example, with SpO2 measurements. This is because reflective PPG sensor technology is sensitive to the contact status between the sensor and human skin, i.e. the exact wearing configuration of the sensor affects the PPG signal quality, thus affecting the vital signs tracking accuracy. Movement and pressure changes are especially likely to cause irregularities/inaccuracies in the measurements of SPO2, for example.

### SUMMARY OF THE INVENTION

The invention is defined by the claims.

According to examples in accordance with an aspect of the invention, there is provided a PPG sensor system, comprising: a first light source configured to emit light; a light detector configured to detect light incident on the light detector; and an optical waveguide comprising a light inlet at a proximal end of the optical waveguide configured to receive emitted light from the first light source, and a light outlet at a distal end of the optical waveguide configured to emit light received via the light inlet.

The invention proposes the use of an optical waveguide to transmit light from a light source to a distal end of the waveguide. In this way, light from the light source can be emitted at a location separate to the location of said light source. The waveguide may only receive part of the emitted light from the light source, or it may receive the entirety of the light emitted from the light source.

By employing an optical waveguide, light can be emitted in a PPG sensor system at a point non-proximate to the light source. For instance, light can be emitted from the waveguide at a point opposite the light detector and pointed towards it, such that transmissive PPG may be enabled. By using an optical waveguide, the light source and the light detector can be integrated in the same circuit board and housing, thus increasing the efficiency of the system.

In other words, by using an optical waveguide, a system capable of transmissive PPG is provided in which the light source and light detector can be integrated in the same circuit board, and thus can be housed together. The housing can be watertight, thus allowing the PPG system to be integrated beneficially into wearable devices, such as a smart sports watch. Further, the lack of electrical connections having to go in or out of the housing allows the system to be made much more resilient against electromagnetic interference.

Embodiments may be based on the realization that a transmissive PPG light source does not actually need to be situated away from the light detector, i.e. opposite the light detector so that the light passes through tissue on its way to the detector. Instead, a waveguide may be used to transmit light from the light source to a point distant from the light detector where it can be emitted from the waveguide's distal end, as if from a light source located at that point. Thus, a configuration that allows transmissive PPG may be provided whilst still having the light source and light detector housed together on the same circuit board.

In summary, the use of an optical waveguide allows light to be emitted from a location without requiring a light source at that point. The waveguide may be flexible so as to allow adjustment of the location of the distal end of the waveguide, in essence, allowing adjustment of the location of a virtual light source. In this way, adjustable transmissive or reflective PPG may be enabled in a system which can also be made water-resistant and electromagnetic-interference resistant. This system may be of particular use integrated into wearable devices, such as a smart watch or a baby monitor, e.g. a baby monitor sock. This system may also be integrated into pulse oximeter devices. A pulse oximeter device utilizing the present invention would be beneficial in environments with strong magnetic fields, such as in an MRI machine.

In some embodiments, the optical waveguide may be flexible so as to permit adjustment of the direction of the light outlet. This allows the waveguide to be easily adjusted, for instance, to flex around a subject's wrist or finger or ankle and still emit light out of its distal end in a beneficial direction.

In some embodiments, the optical waveguide may be configured such that, in use, the received light is emitted out of the light outlet in a direction towards the light detector. This enables transmissive PPG which requires light to be transmitted towards the detector so that the light may be detected. This allows for rigid PPG sensor systems, such as, for example, a rigid bracelet so that the user need not configure the system themselves. Alternatively, if the waveguide is flexible so as to permit adjustment of the direction of the light outlet, the user may be able to adjust the waveguide to ensure that the light is emitted towards the light detector, and the system may then be able to adapt to the different anatomical geometries of different users.

In some embodiments, the system may further comprise a processor configured to generate at least one vital sign parameter value based on light received by the light detector. This allows information contained in the light received by the light detector to be utilized. The use of this system thus provides an effective non-invasive way to generate vital sign parameter values.

In some embodiments, a vital sign parameter value may comprise at least one of: a heart rate value; a respiration rate value; and a pulse oximetry (SpO2) value. These are clinically beneficial vital sign parameters to determine, and are able to be determined based on light detected by a light detector in a PPG sensor system.

In some embodiments, the system may further comprise a housing, wherein the first light source and the light detector are contained within the housing. This increases the efficiency of the system, allowing the first light source and light detector, which can be on the same circuit board, to be housed together. This also allows the system to be completely sealed and/or made watertight, with no electrical connections going in or out. By having no electronic connections leaving the housing, the design can be made much more resilient against electromagnetic interference. Long wires and interfaces are very susceptible to picking up such interference.

In some embodiments, the housing may be a watertight housing. This allows the system to be integrated, for instance, into a smart sports watch device which users may wish to wear while swimming. The system may therefore be configured so as to permit wireless data transfer and charging.

In some embodiments, the system may further comprise a transparent window positioned between the first light source and the light inlet of the waveguide. This allows the waveguide to be located outside of the housing and still receive light from the light source.

In some embodiments, the optical waveguide may comprise at least one of: glass; acrylic; polycarbonate; quartz; plastic; silicone; carbon; and silica. These are materials found to be beneficial in optical waveguides. For instance, the waveguide may be an optical fiber comprising some or all of these materials.

In some embodiments, the first light source may be proximate to the light detector and wherein the optical waveguide may be configured such that, in use, a portion of light emitted by the first light source is not received by the light inlet of the optical waveguide and is suitable for reflective PPG. This allows one light source to be used both for reflective PPG and transmissive PPG. The portion of light not being received by the waveguide can reflect off a surface or shallow tissue of a subject and be used for reflective PPG. The portion of light received by the waveguide can instead be emitted towards the detector, though a subject's tissue, in order to be used for transmissive PPG. This thereby provides an efficient system capable of both reflective and transmissive PPG with only one light source.

In some embodiments, the system may further comprise a second light source configured to emit light having a wavelength within a second wavelength range, wherein the light inlet may be configured to further receive emitted light from the second light source. This allows the waveguide to receive and transmit light from a second light source as well as the first. This can allow for a greater quantity of light to be transmitted via the waveguide.

In some embodiments, the first light source may be configured to emit light having a wavelength within a first wavelength range different to the second wavelength range. This allows the waveguide to receive and transmit light of at least two different wavelengths, for instance, both red and infrared light, which are both beneficial in transmissive PPG.

In some embodiments, the system may further comprise a reflection light source configured to emit light in a reflection wavelength band, wherein the reflection light source may be proximate to the light detector such that, in use, the light detector is able to receive emitted light from the reflection light source reflected off a surface of a subject. This allows a light source more suitable for reflection PPG to be used in the same system as the first light source, which may be configured for transmissive PPG. For example, green light has been found to be beneficial in reflective PPG but is not particularly effective in transmissive PPG. The system may therefore comprise different light sources adapted for different PPG sensing purposes, thereby providing a more effective system.

In some embodiments, the second light source may also be contained within the housing.

In some embodiments, the reflection light source may also be contained within the housing.

In some embodiments, the transparent window may further be configured to be positioned between the second light source and the light inlet.

In some embodiments, the system may comprise a further transparent window configured to be, in use, positioned between the reflection light source and a surface of a subject.

In some embodiments, the system may comprise a further transparent window configured to be, in use, positioned between the light detector and a surface of a subject.

In some embodiments, the further transparent window may be the transparent window.

There is also provided a wearable device comprising any herein disclosed PPG sensor system.

In some embodiments, the wearable device may comprise a smart watch or a baby monitor. For instance, the baby monitor may be a device wearable by the baby such as a smart baby sock or a smart baby glove.

There is also provided a pulse oximeter device comprising any herein disclosed PPG sensor system.

There is also provided a method of PPG sensing, the method comprising: receiving light at a light inlet at a proximal end of an optical waveguide; emitting the received light at a light outlet at a distance end of an optical waveguide; and detecting the emitted received light with a light detector.

Thus, there may be proposed concepts for a PPG sensor system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a simplified block diagram of a PPG sensor system according to a proposed embodiment;
Fig. 2 shows a simplified illustration of a PPG sensor system according to a proposed embodiment;
Fig. 3 shows a simplified view of a PPG sensor system attached to a subject's finger according to a proposed embodiment;
Fig. 4 shows a simplified view of a PPG sensor system attached to a subject's foot according to a proposed embodiment;
Fig. 5 shows a simplified view of a wearable device attached to a subject's limb comprising a PPG sensor system according to a proposed embodiment; and
Fig. 6 shows a flow diagram of a method of PPG sensing according to a proposed embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a PPG sensor system which comprises an optical waveguide, facilitating the transmission of light from a light source to a distal end of the waveguide. In this way, light from the light source can be emitted at a location separate to the location of said light source. The waveguide may only receive part of the emitted light from the light source, or it may receive the entirety of the light emitted from the light source. By employing an optical waveguide, light can be emitted in a PPG sensor system at a point non-proximate to the light source. For instance, light can be emitted from the waveguide at a point opposite the light detector and pointed towards it, such that transmissive PPG may be enabled. By using an optical waveguide, the light source and the light detector can be integrated in the same circuit board and housing, thus increasing the efficiency of the system.

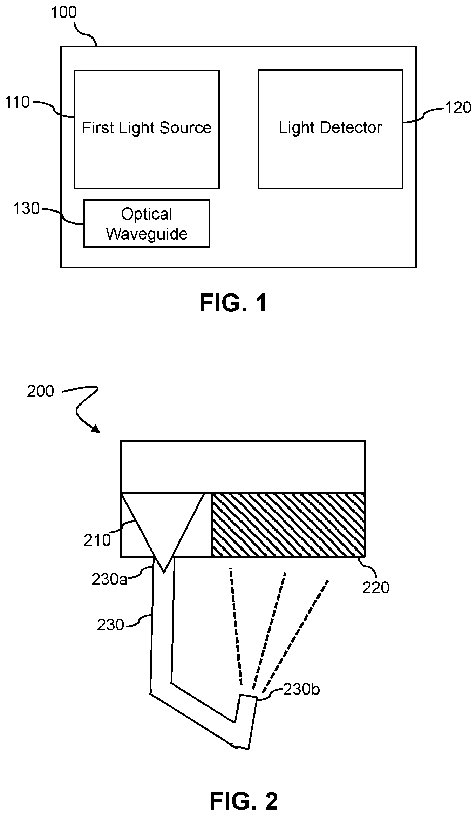

Referring now to Fig. 1, there is depicted a simplified block diagram of a PPG sensor system 100 according to a proposed embodiment. The system 100 comprises a first light source 110 configured to emit light; a light detector 120 configured to detect light incident on the light detector; and an optical waveguide 130 comprising a light inlet at a proximal end of the optical waveguide configured to receive emitted light from the first light source 110, and a light outlet at a distal end of the optical waveguide configured to emit light received via the light inlet.

The light detector 120 may comprise at least one photodiode. The light source 110 may comprise at least one LED. The optical waveguide 130 is an apparatus in which light can enter in one end, and because of refraction against the walls of the waveguide, stay captured and be guided to the other end of the waveguide, at which point it can be emitted. The waveguide 130 therefore essentially allows the system 100 to function as if the first light source 110 were instead located at the distal end of the waveguide.

In essence, the present invention allows the manufacturing of a PPG sensor system that is very similar to a purely reflective PPG setup, wherein the light source(s) and light detector(s) are on the same circuit board, but wherein transmissive PPG sensing is also possible.

Referring now to Fig. 2, there is depicted a simplified illustration of a PPG sensor system 200 according to a proposed embodiment. The system 200 comprises a first light source 210 configured to emit light, and an optical waveguide 230 comprising a light inlet 230a at a proximal end of the optical waveguide configured to receive emitted light from the first light source 210. The waveguide 230 also comprises a light outlet 230b at a distal end of the optical waveguide configured to emit light received via the light inlet 230a.

In this embodiment, the optical waveguide 230 is flexible so as to permit adjustment of the direction of the light outlet 230b. This allows the waveguide to be easily adjusted, for instance, to flex around a subject's wrist or finger or ankle and still emit light out of its distal end 230b in a beneficial direction. The direction of the light outlet 230b can therefore be adjusted to, for instance, ensure that light is emitted towards the light detector 220, thus facilitating transmissive PPG. With a flexible waveguide 230, the system 200 is also more easily able to adapt to the different anatomical geometries of different subjects. It is preferable that the light outlet 230b touches a subject's tissue in order to ensure good transmission of the emitted light through the subject's tissue, and to this end, it is beneficial that the waveguide 230 be flexible so as to permit this in a wide range of circumstances. It is not necessary that the waveguide 230 be flexible, however. For example, the waveguide 230 may be rigid so that a subject or clinician need not configure the system 200 themselves. For instance, the waveguide 230 may be contained within a rigid bracelet that the subject can simply wear.

In this embodiment, the optical waveguide 230 is also configured such that, in use, the received light is emitted out of the light outlet 230b in a direction towards the light detector 220 of the system 200. Preferably, the light outlet 230b should be configured to be at a distance of between 20-35mm away from the center of the light detector 220. These distances are if the waveguide 230 were to be laid out straight in the same plane as the light detector 220, i.e. flat and not bent. Ideally, the distance should be 25mm. In other words, preferably the waveguide 220 should be of a length so that its length plus the length from the center of the light detector 220 to the light inlet 230a adds up to between 20-35mm and ideally 25mm.

In this embodiment, the optical waveguide 230 comprises an optical fiber. The optical fiber comprises glass and plastic. In other embodiments, the optical waveguide 230 may comprise at least one of: glass; acrylic; polycarbonate; quartz; plastic; silicone; carbon; and silica. These are materials found to be beneficial in optical waveguides.

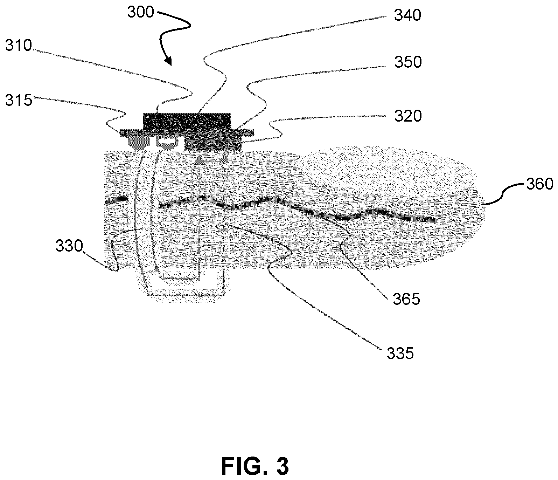

Referring now to Fig. 3, there is provided a simplified view of a PPG sensor system 300 attached to a subject's finger 360 according to a proposed embodiment.

The system 300 comprises a first light source 310, a second light source 315, and a light detector 320. These elements are integrated into the same circuit board 350, which is also in communication with a processor 340. Light from the two light sources, 310 and 315, travels through the optical waveguide 330 and is then emitted from the optical waveguide opposite the light detector 320. The emitted light 335 passes through the subject's finger 360 and through a vein 365 and is then detected by the light detector 320. Transmissive PPG is thus enabled by the system 300. The processor 340 is configured to generate at least one vital sign parameter value based on light 335 received by the light detector 320. This allows information contained in the light received by the light detector to be utilized. The use of this system 300 thus provides an effective non-invasive way to generate vital sign parameter values. A vital sign parameter values comprises at least one of: a heart rate value; a respiration rate value; and a pulse oximetry (SpO2) value. These are clinically beneficial vital sign parameters to determine, and are able to be determined based on light 335 detected by a light detector 320 in a PPG sensor system 300.

In some embodiments, the system 300 may further comprise an analog to digital converter.

In this embodiment, the system 300 comprises a second light source 315 configured to emit light having a wavelength within a second wavelength range, wherein the light inlet of the waveguide 330 is configured to further receive emitted light from the second light source 315. This allows the waveguide 330 to receive and transmit light from a second light source 315 as well as the first 310.

In this embodiment, the first light source 310 is configured to emit light having a wavelength within a first wavelength range different to the second wavelength range. This allows the waveguide 330 to receive and transmit light of at least two different wavelengths, for instance, both red and infrared light, which are both beneficial in transmissive PPG. Typically, PPG measurements of SpO2 make use of the difference between red and infrared light measurements. In this embodiment, the first light source 310 is thus configured to emit light having a wavelength within a first wavelength range of 620nm to 750nm, i.e. red light. The second light source 315 is thus configured to emit light having a wavelength within a second wavelength range of 750nm to 1mm, i.e. infrared light.

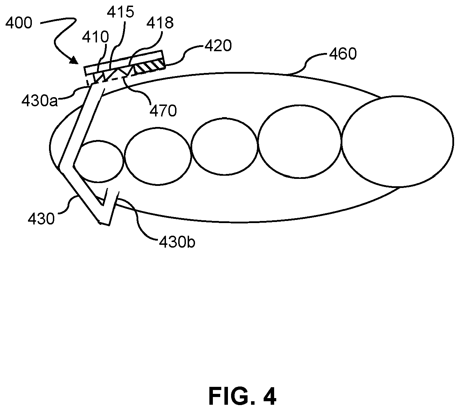

Referring now to Fig. 4, there is depicted a simplified view of a PPG sensor system 400 attached to a subject's foot 460 according to a proposed embodiment. This system 400 may, for example, be integrated into a smart baby sock.

The system 400 comprises a first light source 410 and a second light source 415, much the same as the first and second light sources 310 and 315 described in relation to Fig. 3. The optical waveguide 430 comprising a light inlet 430a and a light outlet 430b is much the same as the waveguides described previously. The system 400 further comprises a reflection light source 418.

The reflection light source 418 is configured for sufficient light output at a distance of between 3.5mm to 5mm from the center of the light detector 420. The distance is preferably 4mm. In other words, the center of the reflection light source 418 should preferably be 3.5mm-5mm laterally away from the center of the light detector 420, and ideally 4mm.

In this embodiment, the system 400 further comprises a housing 470, wherein the first light source 410, second light source 415, reflection light source 418 and the light detector 420 are contained within the housing. This increases the efficiency of the system 400, allowing all the light sources, 410, 415, 418, and light detector 420, which can be on the same circuit board, to be housed together. This also allows the system to be completely sealed and/or easily made watertight, with no electrical connections going in or out. By having no electronic connections leaving the housing, the design is also made more resilient against electromagnetic interference. Long wires and interfaces are very susceptible to picking up such interference. Therefore, by removing any externally placed light sources that would require electrical connections, this point of interference is removed.

In some embodiments, however, the light sources, 410, 415, 418, may not all be in the same housing 470. The light sources may be in separate housings or some light sources may not be in housing at all.

In this embodiment, the housing 470 is a watertight housing. This allows the system 400 to be integrated, for instance, into a smart sports watch device which subjects may wish to wear while swimming. The system 400 may also be integrated into a baby monitor, such as a baby monitor sock or a baby monitor glove. The system 400 may therefore be configured so as to permit wireless data transfer and charging. By having no electrical connections going in or out of the housing, it is much easier and cost effective to seal the housing completely and make it fully waterproof.

The reflection light source 418 is configured to emit light in a reflection wavelength band, wherein the reflection light source is proximate to the light detector 420 such that, in use, the light detector is able to receive emitted light from the reflection light source 418 reflected off a surface or shallow tissue of a subject, such as a vein close to the skin. This allows a light source more suitable for reflection PPG to be used in the same system 400 as the first light source 410 and second light source 415, which are configured for transmissive PPG. For example, green light has been found to be beneficial in reflective PPG, especially in determining pulse rate and respiration rate, but is not particularly effective in transmissive PPG. The system 400 therefore comprises a separate reflection light source 418 with a wavelength in a wavelength range of 495nm to 570 nm, i.e. green light.

In this embodiment, the entire housing 470 is transparent. The term "transparent" refers to a material or substance that allows light to pass through it without significant scattering, absorption, or reflection, such that light can transfer through the material without being significantly affected. In other words, a transparent material is one that is clear or see-through. The degree of transparency can vary depending on the material and its thickness, as well as the properties of the light passing through it, such as wavelength and intensity. Some examples of transparent materials include glass, water, air, and certain plastics.

In some embodiments, the housing 470 may instead be opaque and comprise a transparent window positioned between the light sources, 410, 415, 418, and the light inlet 430a of the waveguide 430. This allows the waveguide 430 to be located outside of the housing and still receive light from the light sources. In some embodiments in which the housing 470 is opaque, the system 400 may further comprise a further transparent window configured to be, in use, positioned between the light detector 420 and a surface of the subject. In some embodiments, the further transparent window may be the transparent window, such that, for example, there is one large transparent window that is proximate to all light sources and the light detector.

This embodiment, along with any other disclosed herein, may form part of a wearable device, such as a smart watch, a sports watch, sleep monitor, or a baby monitor. For instance, the baby monitor may be a device wearable by a baby such as a smart baby sock or a smart baby glove. This embodiment, along with any other disclosed herein, may alternatively form part of a pulse oximeter device. A pulse oximeter device utilizing the present invention would be beneficial in environments with strong magnetic fields, such as in an MRI machine.

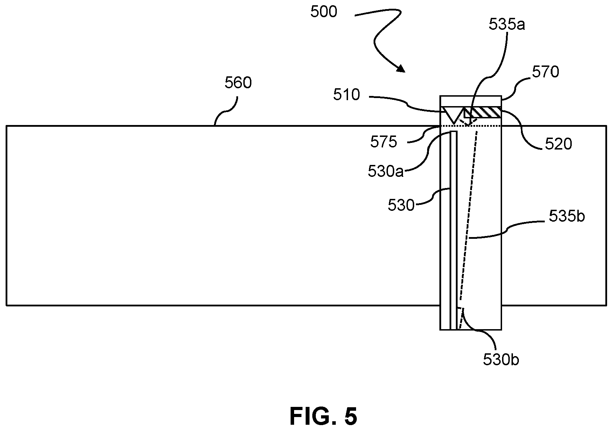

Referring now to Fig. 5, there is depicted a simplified view of a wearable device 500 attached to a subject's limb 560 comprising a PPG sensor system according to a proposed embodiment. The limb 560 may be a subject's arm or leg.

The wearable device 500 comprises housing 570, which itself comprises a transparent window 575. Within the housing is a first light source 510 and a light detector 520. The transparent window 575 is positioned between the first light source 510 and the light inlet 530a of the optical waveguide 530. The transparent window 575 is also positioned between the light detector 520 and a surface of the subject. In some embodiments, the wearable device 500 may be a smart watch with a flexible strap or a rigid bracelet.

In this embodiment, the first light source 510 is proximate to the light detector 520, and the optical waveguide 530 is configured such that, in use, a portion of light 535a emitted by the first light source 510 is not received by the light inlet 530a of the optical waveguide 530 and is suitable for reflective PPG. This portion of light 535a may then reflect off a surface of the subject, e.g. a shallow vein in the subject's limb 560, and be detected by the light detector 520. The remaining portion 535b may then be emitted out of the distal end 530b of the waveguide 530. In other words, this allows one light source 510 to be used both for reflective PPG and transmissive PPG. The portion of light 535a not being received by the waveguide 530 can reflect off a surface of a subject and be used for reflective PPG, and the portion of light 535b received by the waveguide 530 can be emitted towards the detector 520, though a subject's tissue, in order to be used for transmissive PPG. This thereby provides an efficient device 500 capable of both reflective and transmissive PPG with only one light source.

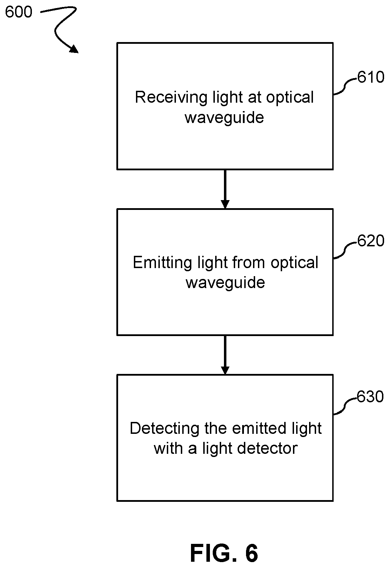

Referring now to Fig. 6, there is provided a flow diagram of a method 600 of PPG sensing according to a proposed embodiment. The method 600 begins with step 610 of receiving light at a light inlet at a proximal end of an optical waveguide. Step 620 comprises emitting the received light at a light outlet at a distance end of an optical waveguide. Finally, step 630 comprises detecting the emitted received light with a light detector. This method may be implemented by any herein disclosed embodiment of a system according to the present invention.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A PPG sensor system (100) comprising:
a first light source (110) configured to emit light;
a light detector (120) configured to detect light incident on the light detector; and
an optical waveguide (130) comprising a light inlet (230a) at a proximal end of the optical waveguide configured to receive emitted light from the first light source, and a light outlet (230b) at a distal end of the optical waveguide configured to emit light received via the light inlet.

2. The system of claim 1, wherein the optical waveguide (130) is flexible so as to permit adjustment of the direction of the light outlet (230b).

3. The system of claim 1 or 2, wherein the optical waveguide (130) is configured such that, in use, the received light is emitted out of the light outlet (230b) in a direction towards the light detector (120).

4. The system of any prior claim further comprising a processor (340) configured to generate at least one vital sign parameter value based on light received by the light detector (120).

5. The system of claim 4, wherein a vital sign parameter value comprises at least one of: a heart rate value; a respiration rate value; and a pulse oximetry (SpO2) value.

6. The system of any prior claim further comprising a housing (570), wherein the first light source (510) and the light detector (520) are contained within the housing.

7. The system of claim 6, wherein the housing (570) is a watertight housing.

8. The system of any prior claim, further comprising a transparent window (575) positioned between the first light source (510) and the light inlet of the waveguide (530a).

9. The system of any prior claim, wherein the optical waveguide (130) comprises at least one of: glass; acrylic; polycarbonate; quartz; plastic; silicone; carbon; and silica.

10. The system of any prior claim, wherein the first light source (110) is proximate to the light detector (120) and wherein the optical waveguide (130) is configured such that, in use, a portion of light emitted by the first light source is not received by the light inlet (230a) of the optical waveguide and is suitable for reflective PPG.

11. The system of any prior claim further comprising a second light source (315) configured to emit light having a wavelength within a second wavelength range, wherein the light inlet is configured to further receive emitted light from the second light source, and preferably wherein the first light source (310) is configured to emit light having a wavelength within a first wavelength range different to the second wavelength range.

12. The system of any prior claim, further comprising a reflection light source (418) configured to emit light in a reflection wavelength band, wherein the reflection light source is proximate to the light detector (420) such that, in use, the light detector is able to receive emitted light from the reflection light source reflected off a surface of a subject.

13. A wearable device (500) comprising a system of any of claims 1 to 12.

14. A pulse oximeter device comprising a system of any of claims 1 to 12.

15. A method (600) of PPG sensing, the method comprising:
receiving light (610) at a light inlet at a proximal end of an optical waveguide;
emitting the received light (620) at a light outlet at a distance end of an optical waveguide; and
detecting the emitted received light (630) with a light detector.
